# EUROPEAN PATENT APPLICATION

(11) **EP 3 827 730 A1**
(43) Date of publication of application: **02.06.2021**
(21) Application number: 20209720.0
(22) Date of filing: 25.11.2020
(51) Int. Cl.: A61B 1/00, A61B 1/018

(54) **ENDOSCOPE DEVICE**

(30) Priority: 26.11.2019 US 201962940673 P
(71) Applicant: Karl Storz SE & Co. KG, 78532 Tuttlingen (DE)
(72) Inventor: BIRNKRANT, Dashiell, 78532 Tuttlingen (DE); BLOMSTROM, Gregory, 78532 Tuttlingen (DE); BROWN, Michael, 78532 Tuttlingen (DE); HOFER, Jochen, 78532 Tuttlingen (DE)

(57) **Abstract**

For the purpose of improve characteristics of an endoscope (10) regarding atraumatic insertion of an endoscopic device (12) into a patient, which especially allows for a compact arrangement of further functional elements, such as a working channel (50), an imaging unit (56), as well as a lighting unit (60). An endoscope device (12) for a flexible endoscope is proposed having at least one endoscope shaft (14) and having at least one distal endoscope tip (18) arranged on the endoscope shaft (14) and which has at least one first section (20) which comprises at least one first contour (22) in the shape of a circle (24) and which has at least one second section (30) which has at least one second contour (32) in the shape of an oval (36), wherein the oval (36) comprises a major axis (38) along which the oval (36) is mirror-symmetrical and a minor axis (40) perpendicular to the major axis (38) along which the oval (36) is mirror-asymmetrical.

## Description

An objective of the invention is to provide a device with improved characteristics regarding atraumatic insertion of an endoscopic device into a patient, which especially allows for a compact arrangement of further functional elements, such as a working channel, an imaging unit, as well as a lighting unit. The objective is achieved, according to the invention, by the features of claim 1, while advantageous implementations and further developments of the invention may be gathered from further dependent claims.

An endoscope device for a flexible endoscope is proposed having at least one endoscope shaft and having at least one distal endoscope tip arranged on the endoscope shaft and which has at least one first section which comprises at least one first contour in the shape of a circle and which has at least one second section which has at least one second contour in the shape of an oval, wherein the oval comprises a major axis along which the oval is mirror-symmetrical and a minor axis perpendicular to the major axis along which the oval is mirror-asymmetrical.

Herein the endoscope device is not to be limited to the application and implementation described above. In particular for the purpose of fulfilling a functionality herein described the endoscope device may comprise a number of respective elements, structural components and units that differs from the number mentioned herein. Furthermore, regarding the value ranges mentioned in this disclosure, values within the limits mentioned are to be understood to be also disclosed and to be used as applicable.

Further advantages may become apparent from the following description of the drawing. In the drawing exemplary embodiments of the invention are shown. The drawing, the description and the claims contain a plurality of features in combination. The person having ordinary skill in the art will purposefully also consider the features separately and will find further expedient combinations.

If there is more than one specimen of a certain object, only one of these is given a reference numeral in the figures and in the description. The description of this specimen may be correspondingly transferred to the other specimens of the object.

It is shown in:
Fig. 1 a schematic illustration of an endoscope with an endoscope device in a perspective view and
Fig. 2 a schematic illustration of a portion of the endoscope device in a front view.

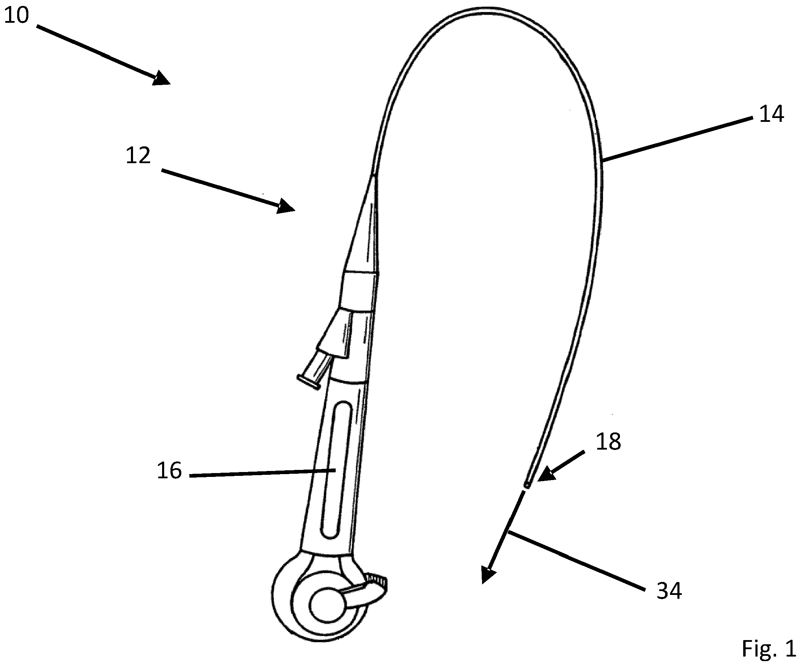

In Fig. 1 a schematic illustration of an endoscope 10 is shown. The endoscope 10 is implemented as a flexible endoscope. The endoscope 10 comprises an endoscope device 12. The endoscope device 12 may only embody a portion of the endoscope 10. In the current embodiment the endoscope device 12 embodies the entire endoscope 10. The endoscope device 12 comprises at least one endoscope shaft 14. The endoscope shaft 14 is flexibly implemented. The endoscope shaft 14 is connected to a handle 16 of the endoscope device 12.

The endoscope device 12 comprises at least one distal endoscope tip 18. The distal endoscope tip 18 is arranged on the endoscope shaft 14. The distal endoscope tip 18 has at least one first section 20. The first section 20 comprises at least one first contour 22. The first contour 22 has the shape of a circle 24. A front 26 of the distal endoscope tip 18 is flat. The front 26 of the distal endoscope tip 18 is arranged at least substantially perpendicular to a central axis 28 of the distal endoscope tip 18.

The distal endoscope tip 18 has at least one second section 30. The second section 30 forms the front 26 of the distal endoscope tip 18. The second section 30 has at least one second contour 32. Along a distal direction 34 the first section 20 and the shape of its first contour 22 continuously merge into the second section 30 and the shape of its second contour 32. The second contour 32 has the shape of an oval 36. The oval 36 comprises a major axis 38. Along the major axis 38 the oval 36 is mirror-symmetrical. The second contour 32 has a minor axis 40. The minor axis 40 is perpendicular to the major axis 38 along which the oval 36 is mirror-asymmetrical.

The minor axis 40 divides the second section 30 into a first area 42 and a second area 44. The first area 42 has a first minimum radius of curvature 46. The second area 44 has a second minimum radius of curvature 48. The second minimum radius of curvature 48 is different from the first radius of curvature 46. The first minimum radius of curvature 46 is larger than the second minimum radius of curvature 48.

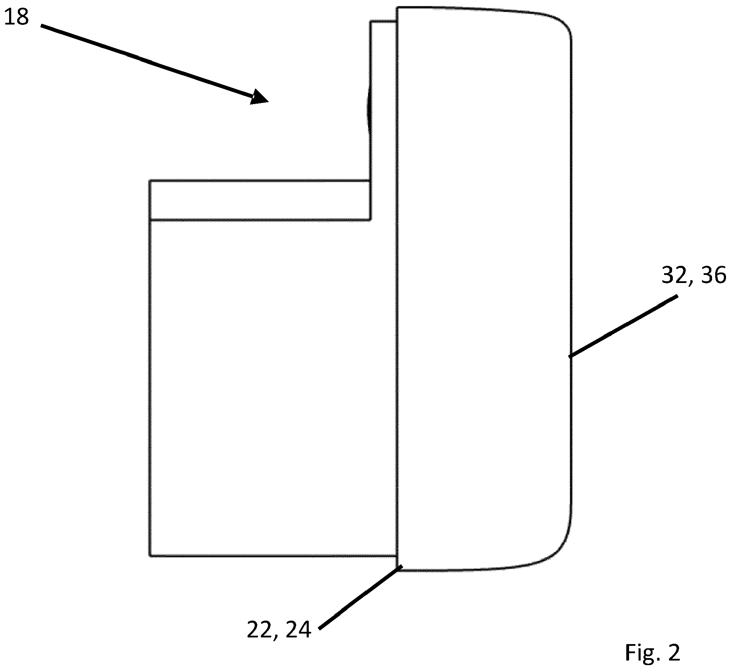

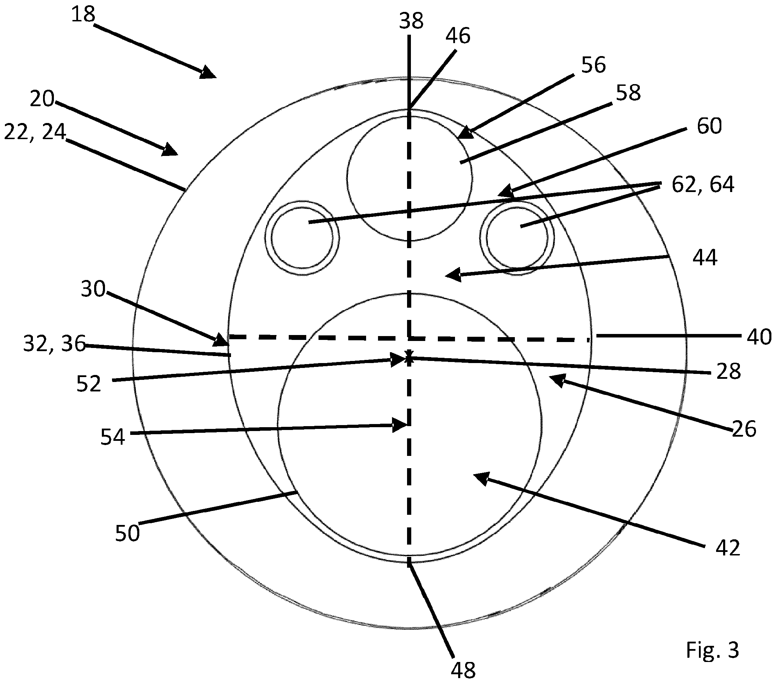

Fig. 2 shows a frontal view of a portion of the endoscope device 12. The endoscope device 12 further comprises a working channel 50. The working channel 50 passes through the endoscope shaft 14. In a frontal view the working channel 50 is arranged at least for the most part in the first area 42 of the second section 30. In a frontal view the working channel 50 is arranged overlapping with a circle center 52 of the first section 20. A geometrical center 54 of the working channel 50 is different from the circle center 52 of the first section 20. In the frontal view the working channel 50 in the second section 30 is mirror-symmetrically intersected by the major axis 38.

The endoscope device 12 further comprises an imaging unit 56. The imaging unit 56 may pass through the endoscope shaft 14. For example, the imaging unit 56 may comprise optical fibers which may extend through the shaft 14. In the current embodiment the imaging unit 56 comprises an imaging sensor 58 such as a CCD-sensor, a CMOS-sensor or the like, which is arranged at least partially in the distal endoscope tip 18. In a frontal view the imaging unit 56 is arranged at least for the most part in the second area 44 of the second section 30. In a frontal view the imaging unit 56 is mirror-symmetrically intersected by the major axis 38 in the second section 30.

The endoscope device 12 further comprises a lighting unit 60. The lighting unit 60 may pass through the endoscope shaft 14. For example, the lighting unit 60 may comprise optical fibers which may extend through the shaft 14. In the current embodiment the lighting unit 60 comprises at least one light source 64 such as a LED, OLED or the like, which is arranged at least partially in the distal endoscope tip 18. In a frontal view the lighting unit 60 is arranged at least for the most part in the second area 44 of the second section 30. The lighting unit 60 comprises at least two lighting elements 62, 64. The lighting elements 62, 64 may each embody a light source. Alternatively, the lighting elements 62, 64 may embody optical fibers. The two lighting elements 62, 64 are arranged flanking the image acquisition unit. In a frontal view the lighting elements 62, 64 are arranged mirror-symmetrically to one another with respect to the major axis 38.

### Reference numerals

- 10: Endoscope
- 12: Endoscope device
- 14: Endoscope shaft
- 16: handle
- 18: distal endoscope tip
- 20: First section
- 22: First contour
- 24: circle
- 26: front
- 28: Central axis
- 30: Second section
- 32: Second contour
- 34: Distal direction
- 36: Oval
- 38: Major axis
- 40: Minor axis
- 42: First area
- 44: Second area
- 46: first minimum radius of curvature
- 48: second minimum radius of curvature
- 50: Working channel
- 52: circle center of the first section
- 54: geometrical center
- 56: Imaging unit
- 58: Image sensor
- 60: Lighting unit
- 62: Light source
- 64: Lighting elements

## Claims

1. Endoscope device (12) for a flexible endoscope having at least one endoscope shaft (14) and having at least one distal endoscope tip (18) arranged on the endoscope shaft (14) and which has at least one first section (20) which comprises at least one first contour (22) in the shape of a circle (24) and which has at least one second section (30) which has at least one second contour (32) in the shape of an oval (36), wherein the oval (36) comprises a major axis (38) along which the oval (36) is mirror-symmetrical and a minor axis (40) perpendicular to the major axis (38) along which the oval (36) is mirror-asymmetrical.

2. Endoscope device (12) according to claim 1, wherein along a distal direction (34) the first section (20) and the shape of its first contour (22) continuously merge into the second section (30) and the shape of its second contour (32).

3. Endoscope device (12) according to claim 1 or 2, wherein the second section (30) forms a front (26) of the endoscope tip (18).

4. Endoscope device (12) according to claim 3, wherein the front (26) of the distal endoscope tip (18) is flat and/or is arranged at least substantially perpendicular to a central axis (28) of the distal endoscope tip (18).

5. Endoscope device (12) according to one of the preceding claims, wherein the minor axis (40) divides the second section (30) into a first area (42) having a first minimum radius of curvature (46) and a second area (44) having a second minimum radius of curvature (48), wherein the first minimum radius of curvature (46) is larger than the second minimum radius of curvature (48).

6. Endoscope device (12) according to claim 5, comprising a working channel (50) which passes through the endoscope shaft (14) and in a frontal view is arranged at least for the most part in the first area (42) of the second section (30).

7. Endoscope device (12) according to claim 6, wherein in a frontal view the working channel (50) is arranged overlapping with a circle center (52) of the first section (22), a geometrical center (54) of the working channel (50) is different from the circle center (52) of the first section (22).

8. Endoscope device (12) according to claim 6 or 7, wherein in a frontal view the working channel (50) in the second section (30) is mirror-symmetrically intersect by the major axis (38).

9. Endoscope device (12) according to claim 5, comprising an imaging unit (56) which passes through the endoscope shaft (14) and/or which is arranged at least partially in the distal endoscope tip (18) and in frontal view is arranged at least for the most part in the second area (44) of the second section (30).

10. Endoscope device (12) according to claim 9, wherein in a frontal view the imaging unit (56) in the second section (30) is mirror-symmetrically intersect by the major axis (38).

11. Endoscope device (12) according to claim 5, comprising a lighting unit (60), which passes through the endoscope shaft (14) and/or which is arranged at least partially in the distal endoscope tip (18) and in frontal view is arranged at least for the most part in the second area (44) of the second section (30).

12. Endoscope device (12) according to claim 11, wherein the lighting unit (60) comprises at least two lighting elements (64) which in a frontal view are arranged mirror-symmetrically to one another with respect to the major axis (38).

13. Endoscope device (12) according to claims 9 and 12, wherein the two lighting elements (64) are arranged flanking the imaging unit (56).

14. Endoscope (10) with at least one endoscope device according to one of the preceding claims.
